(19) Europäisches Patentamt
European Patent Office
Office européen des brevets

(11) EP 3 730 051 A1

(12) EUROPEAN PATENT APPLICATION

(43) Date of publication:
28.10.2020 Bulletin 2020/44

(21) Application number: 20175066.8

(22) Date of filing: 27.11.2018

(51) Int Cl.:
A61B 5/06 (2006.01)          A61B 5/00 (2006.01)
A61G 13/10 (2006.01)         A61G 13/12 (2006.01)
A61B 34/20 (2016.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 28.11.2017 US 201762591238 P
12.09.2018 US 201816129263

(62) Document number(s) of the earlier application(s) in
accordance with Art. 76 EPC:
18208600.9 / 3 488 779

(71) Applicant: Biosense Webster (Israel) Ltd
Yokneam, 2066717 (IL)

(72) Inventors:
• DEMRI, Tamir
2010300 Gilon (IL)
• ELDAR, Ranit
4645511 Herzliya (IL)
• BLATT, Ofir
2018100 Oranit (IL)
• MONTAG, Avram Dan
2066717 Yokneam (IL)

(74) Representative: Small, Gary James
Carpmaels & Ransford LLP
One Southampton Row
London WC1B 5HA (GB)

Remarks:
This application was filed on 15.05.20 as a divisional
application to the application mentioned under INID
code 62.

(54) **LOW PROFILE DUAL PAD MAGNETIC FIELD LOCATION SYSTEM WITH SELF TRACKING**

(57)     A magnetic field pad location system is provided with dual elongated field generation pads with self-tracking. Each pad comprises magnetic coils operable to generate magnetic fields. The pads include a mechanism for securing the two pads in place on a patient table to prevent movement of the pads. A processor executes a process for installation of the two pads, comprising correlation of the magnetic fields generated by the coils to define a reference magnetic field for sensing. In one embodiment, each pad includes a same number of magnetic coils in each spatial direction. In one embodiment, the magnetic coils are tri-axial coils placed at corners of the pads. In another embodiment, each pad includes an array of six single coils.

FIG. 2A

**Description**

**CROSS REFERENCE TO RELATED APPLICATION**

The present application is a non-provisional of Provisional Application No. 62/591,238 filed November 28, 2017 which is incorporated herein by reference as if fully set forth.

**SUMMARY**

A magnetic field location system and method are provided for use in tracking a distal end of a catheter or the like in a surgical or exploratory procedure within a living body disposed on a support structure, such as a patient bed, table or gurney. Dual low-profile elongated field generation pads are provided that are configured for selective placement along respective sides of the living body disposed on the support structure. Each field generation pad includes a plurality of magnetic coils operable to generate magnetic fields.

A securing structure is provided to releasably secure the dual field generation pads to the support structure when selectively placed on the support structure. The dual field generation pads and associated securing structure are preferably configured such that the dual field generation pads are able to be selectively placed on and secured to the support structure in less than 60 seconds and are also able to be removed from the support structure in less than 60 seconds. Preferably, each of the first and second elongated field generation pads are configured with a low-profile height not exceeding 20 mm and a weight not exceeding 3 Kg.

A processor is coupled to the magnetic coils of the dual field generation pads. The processor is configured to execute a process for correlation of magnetic fields generated by the field generation pads such that a reference magnetic field is defined that extends within a desired portion of the living body disposed on the support structure. This enables precise location and tracking of a distal end of a catheter via a magnetic sensor of the catheter within the reference magnetic field.

The dual field generation pads are preferably relatively symmetric and define relatively symmetric magnetic coil arrays when placed by the sides of the living body. In one embodiment, the magnetic fields generated by the dual field generation pads are generated by two magnetic coils disposed in each field generation pad at a defined distance from each other. In such an embodiment, each magnetic coil can be configured as a tri-axial coil unit, such as tri-coil bobbins that have a combined weight of approximately 200 grams. In another embodiment, the magnetic fields generated by the dual field generation pads are generated by six linearly aligned and equally spaced magnetic coils disposed in each field generation pad, where each magnetic coil is configured as a single coil unit.

To secure each field generation pad to the support structure, two securing mechanisms can be attached to each field generation pad at predefined locations spaced apart from each other. In such case, each securing mechanism can have an arm having an end attached to the respective field generation pad and a support structure engaging portion. The support structure engaging portion of the arm can be configured such that the support structure engaging portion engages a side of the support structure when the respective field generation pad is selectively placed on the support structure, thereby defining the relative placement of the respective field generation pad on the support structure.

The pad attaching end of the arm of each such securing mechanism may have an adjustable coupling to enable the positioning of the respective support structure engaging portion at an extended or a retracted position relative to the respective field generation pad. As such, the dual field generation pads are placed furthest from each other and closest to the sides of the support structure when all of the support structure engaging portions are in the retracted position and are placed closest to each other and furthest from the sides of the support structure when all of the support structure engaging portions are in the extended position.

In one embodiment, each securing mechanism has a clamp configured to be operated to a clamping position from an open position. The clamps are preferably operated when the respective support structure engaging portions are engaged with respective sides of the support structure so that field generation pads are secured in their selectively placed positions on the support structure. In another embodiment, each securing mechanism may have a coupling component configured for connection beneath the support structure. In such case, the securing structure can include a first joining member configured to attachment to the coupling components of the first securing mechanisms and a second joining member configured for attachment to the coupling components of the second securing mechanism to secure the selective placement of the field generation pads on the support structure when the joining member are attached.

These and other objects, features and advantages of the present invention will be apparent from the following detailed description of illustrative embodiments thereof, which is to be read in connection with the accompanying drawings.

**BRIEF DESCRIPTION OF THE DRAWINGS**

The present invention is illustrated by way of example, and not by way of limitation, in the figures of the accompanying drawings.

Figure 1 is example schematic, pictorial illustration of a magnetic location system for use in tracking a distal end of a catheter of the like within a living subject during a medical procedure.

Figure 2A is a perspective view of an embodiment the magnetic location system comprising dual low-profile elongated field generation pads secured to a patient table.

Figure 2B is a top view of the dual low-profile elongated field generation pads shown in Fig. 2A.

Figure 2C is a bottom view of the embodiment of the magnetic location system shown in Fig. 2A.

Figure 3 is a perspective view of the of magnetic location system shown in Fig. 2A with an installable arm rest.

Figure 4A is a top view of another embodiment of one of the dual low-profile elongated field generation pads shown in Fig. 2A illustrating an array of rod-connected field generating coil units in phantom.

Figure 4B is a cross section of the low-profile elongated field generation pad along line 4B of Fig. 4A.

Figure 5A is a perspective view of the securing mechanism for the dual low-profile elongated field generation pads of Fig. 2A in an "open" position.

Figure 5B is a side view of the securing mechanism for the dual low-profile elongated field generation pads of Fig. 2A in the "open" position.

Figure 6A is a perspective view of the securing mechanism for the dual low-profile elongated field generation pads of Fig. 2A in a "clamping" position.

Figure 6B is a side view of the securing mechanism for the dual low-profile elongated field generation pads of Fig. 2A in the "clamping" position.

Figure 7 is a bottom view of a portion of one of the dual low-profile elongated field generation pads of Fig. 2A.

Figure 8A is an end view of another embodiment of a securing mechanism for the dual low-profile elongated field generation pads of Fig. 2A.

Figure 8B is a bottom perspective view of the securing mechanism for the dual low-profile elongated field generation pads of Fig. 8A.

Figure 9 is a bottom view of another alternative of a securing mechanism for the dual low-profile elongated field generation pads of Fig. 2A.

## DETAILED DESCRIPTION

Various methods and systems are known in the art for tracking the coordinates of objects involved in medical procedures. For example, magnetic navigation systems may be used for navigating tools, such as medical implements, catheters, wireless devices, etc. One such magnetic navigation system can be found in the CARTO® system, produced by Biosense Webster, Inc. (Diamond Bar, California).

In magnetic navigation systems, magnetic fields are, for example, generated by a location pad that includes a plurality of magnetic field generators. In a magnetic navigation system, magnetic position sensing may be used to determine position coordinates of the distal end of a tool inside a patient's organ. For this purpose, a driver circuit in a console or a location pad drives the plurality of field generators to generate magnetic fields that extend within the body of a patient.

Typically, the field generators comprise a plurality of coils, which are, for example in the CARTO™ system, incorporated into a unitary location pad that is placed below the patient's torso at known position external to the patient, such as below the patient table. The unitary pad system was a significant improvement over the direct application of individual magnetic field generation coils to a patient's torso such as disclosed in U.S. Pat. No. 6,618,612, where coil placement was more cumbersome, could interfere with a performance of a desired medical procedure, and could require disinfectant cleaning for re-use.

The coils generate magnetic fields in a predefined working volume that contains the patient's organ to be explored. One or more magnetic field sensors located on a medical tool detect the magnetic fields and generate electrical signals in response to these magnetic fields. A signal processor processes these signals in order to determine the position coordinates of the medical tool, typically including both location and orientation coordinates. Each coil transmits at a different frequency so that the processor can separate the magnetic fields from each source. This method of position sensing is implemented in the above-mentioned CARTO™ system and is described in detail in U.S. Pat. Nos. 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all incorporated herein by reference.

The CARTO® system has conventionally employed a unitary location pad for generating the magnetic field used for magnetic location sensing. The unitary pad contains multiple magnetic coils that create a magnetic field and is generally placed beneath the patient. The unitary pad generates an ultralow magnetic reference field from which the location of the tool (having a magnetic field sensor) can be determined. This provides the physician with visibility and orientation of the patient's internal anatomy. The construction of suitable magnetic coils, i. e. reference field transducers, is disclosed, for example, in U.S. Pat. No. 6,618,612.

The inventive location system described herein features magnetic field generator coil units 201 disposed in dual low-profile elongated field generation pads 202 that are easier to install on the top of a patient table, such as a bed or gurney 28, on either side of the patient's torso, than currently existing unitary pad or individual field generator systems. The system also includes a self-tracking algorithm to define the magnetic reference field used by a catheter sensor when implementing location sensing and to make installation easier. An example, self-tracking algorithm for the pads is discussed below.

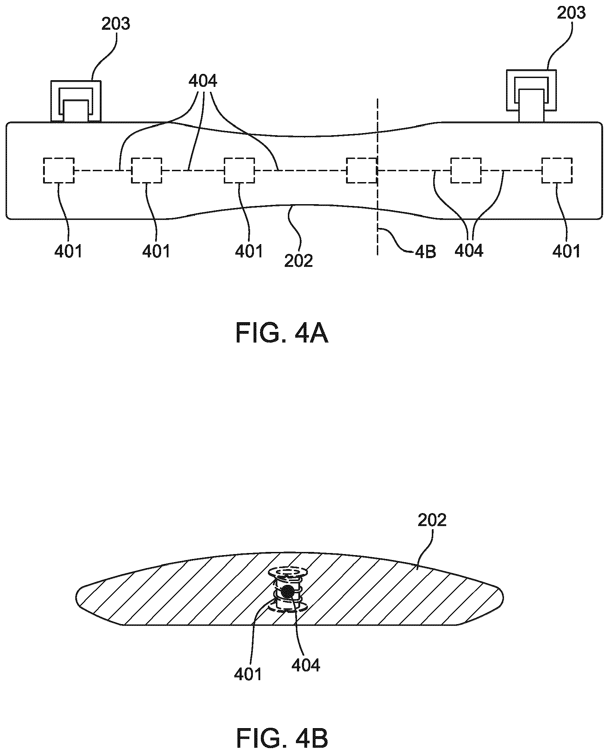

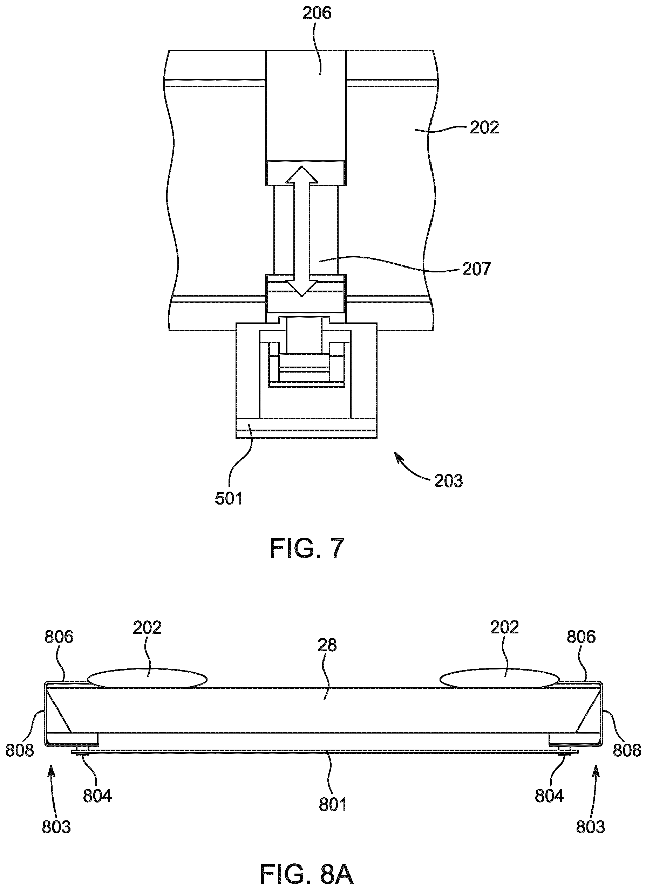

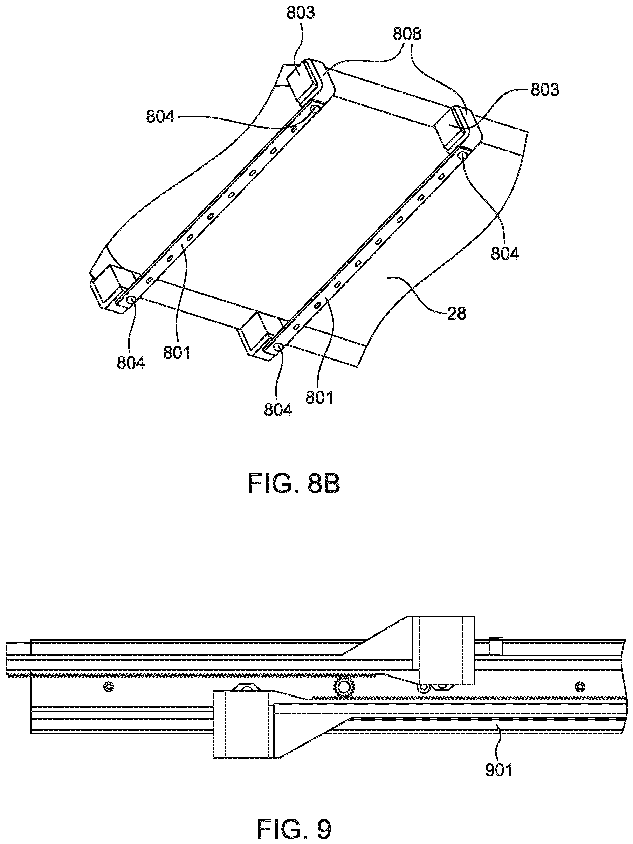

A clamping mechanism, such as clamps 203 of Figs. 4A-7, can be used to secure the field generation pads 202 in place for use. As an alternative, a flexible band, such as elastic member 801 of Fig. 8A-B, can be used for securing the field generation sensor pads 202 on a patient's bed. In lieu of the elastic members 801, a rack and pinion mechanism 901, such as illustrated in Fig. 9, may be employed.

In particular, a first embodiment employs dual low-profile elongated field generation pads 202 rather than the triangular location pad currently used in the CARTO™ system or individual coil units. The dual low-profile elongated field generation pads 202 are preferably situated on an axis, such as the longitudinal axis, of the patient table 28 on either side of a patient and secured in place by a securing structure attach them to the patient table 28. The securing structure is designed to prevent the dual low-profile elongated field generation pads 202 from moving during the medical or exploratory procedure.

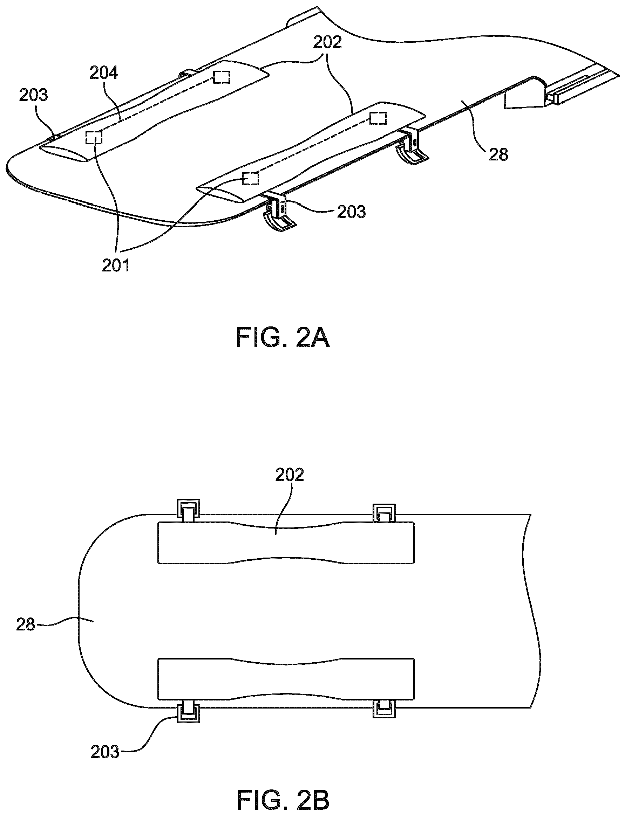

Clamping mechanisms 203 are provided as the securing structure for the embodiment illustrated in Figs 2A-7. The clamping mechanisms 203 allow a physician and/or location pad installer to add the dual low-profile elongated field generation pads 202 before the procedure starts and/or during the procedure. Thus, the patient barely needs to move to add the dual low-profile elongated field generation pads 202 whereas the known unitary location pad and individual coil configurations require considerable effort to allow the location pad or individual coil units to be appropriately positioned. Situating the dual low-profile elongated field generation pads 202 on the table 28 permits the field generating coils 201 to be close to the patient to generate magnetic field gradients for assuring location accuracy and to be distanced from items such as fluoroscopy system collimators which are undesired metal sources that interfere with the magnetic location system.

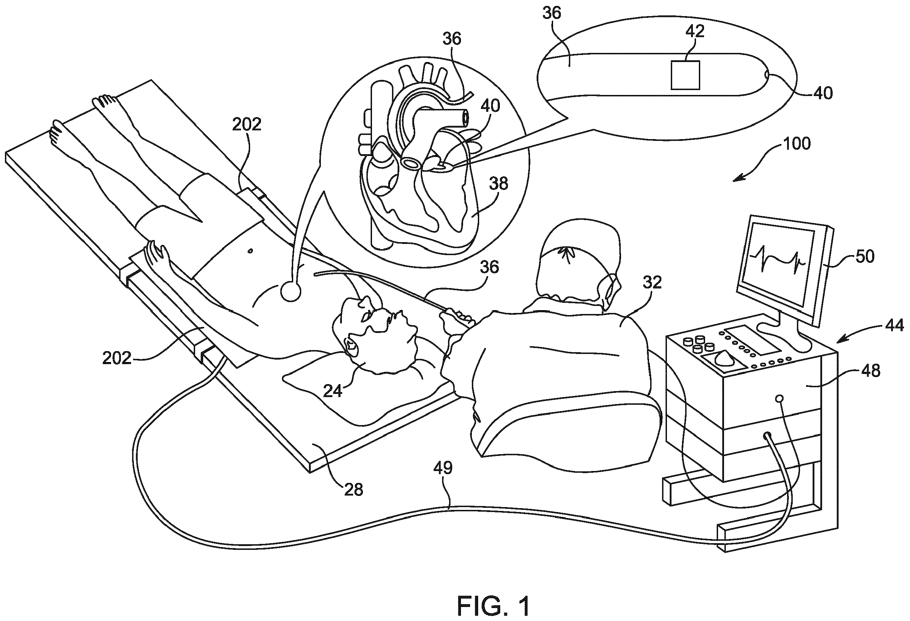

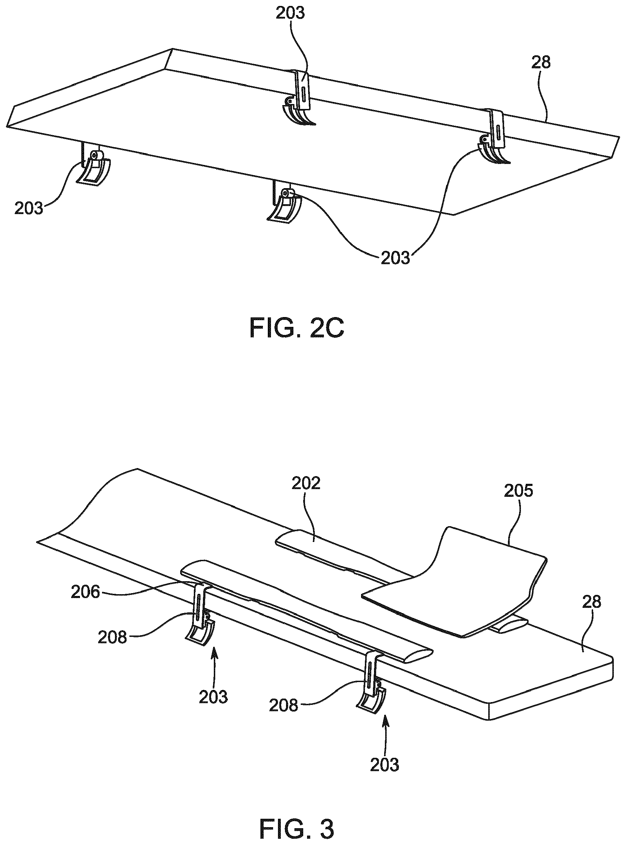

Figure 1 is a schematic, pictorial illustration of a magnetic position tracking system 100 used in cardiac catheterization applications, in accordance with an embodiment of the present invention. System 100 comprises dual low-profile elongated field generation pads 202, which comprises one or more field generators, such as field generating coils. Embodiments of the dual low-profile elongated field generation pads 202 are described in more detail with respect to Figures 2-9.

A patient 24 lies on a catheterization or patient table 28. A physician 32 inserts a catheter 36 into a chamber of a heart 38 (shown as balloon insert) of the patient 24. System 100 determines and displays the position and orientation coordinates a distal end 40 of catheter 36 inside the heart 38.

In the exemplary configuration of Figure 1, the dual low-profile elongated field generation pads 202 comprise embedded field generating coil units 201, 401 see Figures 2A, 4A and 4B. The dual low-profile elongated field generation pads 202 are placed on top of the table 28 along or under the patient's torso 24, such that the coils 201 become located in fixed positions external to the patient 24 and are operable to generate magnetic fields in a defined working volume around the heart. A field definition algorithm is implemented once the pads are positioned which defines the magnetic field for sensor use.

A position sensor 42 fitted in the distal end 40 of the catheter 36 senses the magnetic fields in its vicinity. The position sensor 42 produces and transmits, in response to the sensed fields, position signals to a console 44. The console 44 comprises a tracking processor 48 that calculates the location and orientation of catheter 36 with respect to the coils 201, based on the position signals sent by position sensor 42. Typically, the console 44 also drives the coils 201 through a connector cable 49 to generate the appropriate magnetic fields and to implement the field definition algorithm. The location and orientation coordinates of catheter 36 are displayed to the physician using a display 50.

Although Figure 1 shows an example system 100 for cardiac catheterization or other procedures, the dual low-profile elongated field generation pads 202 can be used in other position tracking applications, such as for tracking orthopedic implants and other medical tools.

The dual low-profile elongated field generation pads 202 may also be compatible with fluoroscopy systems used by the magnetic tracking system. Accordingly, in one embodiment, the dual low-profile elongated field generation pads 202 may be radiolucent when centered in a horizontal plane below a Fluoro iso-center for a Fluoro C-arm orientation of LAO60 - RAO60 (@CRA/CAU 0). Also, the dual low-profile elongated field generation pads 202 may be designed to minimize interference with the Fluoro C-arm movement (considering LAO-RAO rotation). The dual low-profile elongated field generation pads 202 may be robust and flexible to withstand mechanical shocks, such as a drop of up to one meter onto a hard surface (e.g., floor), without damage.

The functional interface of the dual low-profile elongated field generation pads 202 with the tracking processor 48 of the system 100 may be implemented through a wireless connection. In that case, the location pad will be connected only to a power supply through a power cable in lieu of the connector cable 49. The wireless connection can be based on standard wireless protocols (such as WLAN IEEE802.11) + implementation for synchronization.

Figure 2A illustrates example dual low-profile elongated field generation pads 202 positioned along the longitudinal axis of the patient table 28. In this example, the dual low-profile elongated field generation pads 202 have four tri-coils magnetic field generation units 201, two for each pad 202. For each pad, the field generation units 201 are disposed at opposite ends the pad 202 at a fixed distance such that the pad's two field generation coil units 201 are preferably spaced no greater than 500 mm from the center of each other. The distance is selected to assure sufficient magnetic field generation using relatively low profile field generation coil units 201. To maintain the fixed spacing of the coil units within each pad, a connecting rod 204 can be provided having ends secured to each field generation coil unit 201 within the pad 202.

In the Figure 2A example, the field generation coil units 201 are preferably tri-coil bobbins that weigh about 200 grams together so that each pad weighs only 1-2 Kg and is 540 mm in length, 140 mm in width and 8.5 mm in height. As such, the dual low-profile elongated field generation pads 202 are easy to install, have a low profile and low weight compared to existing systems. To permit relatively easy handling and placement of the pads 202, they generally do not exceed 3 Kg in weight and do not have a height exceeding 20 mm.

The dual low-profile elongated field generation pads 202 are installed to produce a magnetic field that defines a desired magnetic field zone in the system 100. In above embodiment, the distance between the dual low-profile elongated field generation pads 202 when installed preferably does not exceed 50 cm to maintain sufficient magnetic field gradients within the working volume.

In connection with installing the pads 202, the tracking processor 48 can be programmed to detect if the pads 202 are positioned within a predetermined maximum distance, such as 50 cm, of each other. The processor can generate a signal that is relayed to the console to display an indication on the display 50 as to whether the pads 202 are positioned close enough to each other to properly generate the magnetic sensing field.

Preferably the pads are symmetric to each other with each having a same number of field generation coil units in each spatial direction, typically multiples of three, are used. The dual low-profile elongated field generation pads 202 may support Improved "Single Axis Sensor" (SAS) accuracy. In contrast to the Figure 2A embodiment, where tri-axial coils 201 are disposed at each end of each of the pads 202, Figs. 4A and 4B illustrates in another embodiment, wherein an array of six single axis coils 401 are equally distributed along the length of each of the dual low-profile elongated field generation pads 202. Connecting rod members 404 are provided to maintain an equal fixed spacing between the single axis field generation coils 401 within each pad 202.

The dual low-profile elongated field generation pads 202 include a securing mechanism, such as clamps 203, to secure the pads 202 to the patient table 28 to prevent unintentional movement of the pads 202 such as a result of lateral force lower than about 100 Newtons. In one embodiment, the dual low-profile elongated field generation pads 202 have an ergonomic design that is in line with the design elements of the entire system to emphasize the fact that dual low-profile elongated field generation pads 202 are parts of one system 100.

The securing structure, such as clamp mechanisms 203, is preferably designed to permit installation of the dual low-profile elongated field generation pads 202 within one minute and uninstallation in even less time. In busy surgical practices, patient surgical operations, mappings and/or other procedures are often performed on a relatively tight time schedule. The present invention greatly facilitates the efficient conduct of such successive procedures by permitting the very rapid uninstallation of the field generation pads from the table of a patient who had undergone a procedure and installation of the pads on the table of another patient scheduled for a next procedure.

The location pad 20 may comprise a holding structure that avoids interference with a patient table arm rest 205 on the patient table 28. As shown in Figure 3, in one embodiment, the field generation pad 202 may be integrated with the arm rest 205 as a single device.

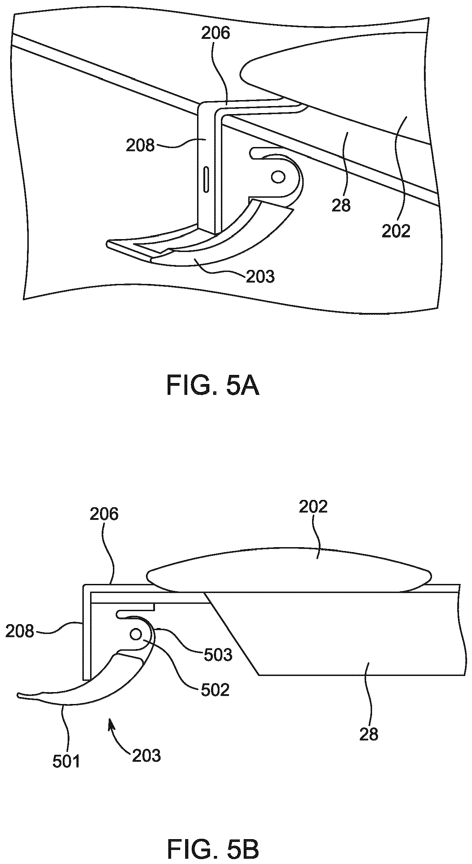

Figures 2A-7 illustrate a simple clamp type mechanism 203 for securing the low-profile elongated field generation pads 202 to the patient table 28. In this example, each pad is provided with two clamp mechanisms 203. As best seen in Figures 5A-6B, the securing clamp mechanisms 203 each include a pad attaching arm 206 connected to a respective field generation pad 202. The arms 206 each have a table engaging portion 208 which is configured to abut against a side of the patient table 28 when the pad 202 is installed so that the engagement of the table engaging portions 208 define the location of the pads 202 relative to the table 28, as best seen in Figures 2A and 2B.

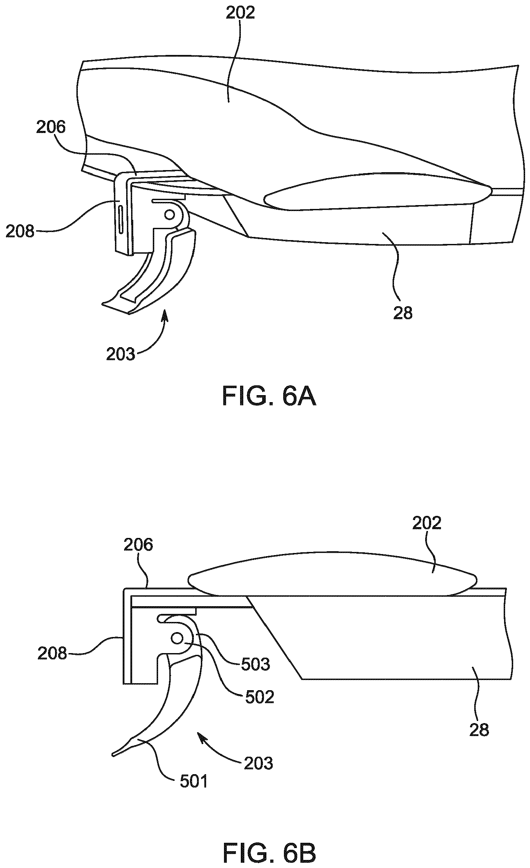

Figures 5A and 5B illustrate views of one of the clamping mechanisms 203 in an open state that enables the field generation pad 202 to be positioned on the table 28 so that the table engaging portion 208 of each clamp mechanism 203 abuts a respective side of the table 28. Figures 6A and 6B illustrate views of the clamping mechanism 203 in a clamping state with the field generation pad 202 appropriately positioned on the table 28.

The example clamping mechanisms 203 are of a lever type design, each having a handle portion 501 that can be easily gripped to toggle between the open and clamping positions by an individual installing the field generation pad 202. The lever handle 501 of the clamping mechanism 203 pivots about a pivot shaft 502 to displace a clamping end portion 503 between clamping and open positions. As shown in Figures 6A and 6B, the handle 501 has been pushed downward which pivots the clamping end portion 503 upwards against the bottom of the patient table. In this clamping position, enough force is exerted on the patient table so that the attached field generation pad 202 is not easily moveable.

Figure 7 shows a bottom view of a portion of one of the field generation pads 202 illustrating the optional adjustability of the attachment of the arm 206 of the clamping mechanism 203 to the pad 202. The arm 206 is equipped with an adjustable coupling 207 which allows adjustment of the distance the table engaging portion 208 from the field generation pad 202. The adjustable coupling 207 can be configured, for example, as a slot/tab mechanism, a slide type mechanism, or projecting pins that are engageable in a variety of locations with respect to a series of defined holes.

The adjustable coupling 207 enables the table engaging portion 208 of the arm 206 of the securing mechanism 203 to be positioned at an extended position or a retracted position relative to the pad 202 and may be configured to permit the attachment of the arm 206 to the table engaging portion 208 at one or more positions therebetween. When installed on the table 28, the dual field generation pads 202 will be furthest from the respective sides of the table 28 and closest to each other when the table engaging portions 208 of the securing mechanisms 203 are in the extended position. When the table engaging portions 208 of the securing mechanisms 203 are in the retracted position, the dual field generation pads 202 will be closest to the respective sides of the table 28 and furthest from each other when installed. The adjustability of the coupling of the securing mechanisms 203 to the pads 202 enables use of the pads with both wide and narrow tables.

Figures 8A and 8B illustrate an alternative embodiment of a table securing mechanism 803 for the field generation pads 202. The securing mechanisms 803 each include a pad attaching arm 806 along with a table engaging portion 808 similar to the arm 206 and table engaging portion 208 of the clamping mechanism 203 described above. In addition, each securing mechanism 803 includes a boss 804 mounted on a portion of the arm 806 that extends to the underside of the table 28. The boss 804 is configured to receive an end of an elastic member or the like 801. The elastic member 801 serves to secure the field generation pads 202 in position on the patient table 28. The arm 806 may include an adjustable coupling, such as arm coupling 207 of clamping mechanism 203, to accommodate different table widths when the securing mechanisms 803 are used. Elastic member 801 may be made of rubber or other elastic material of appropriate length to exert a sufficient securing tension to prevent inadvertent movement of the pads 202 when installed on the patient table 28.

As shown in the example illustrated in Fig. 8B, when the dual field generation pads 202 are installed, each pad 202 is secured to the table by two securing mechanisms 803 which each oppose a respective securing mechanism 803 of the other pad 202. Two elastic members 801 are used, each one securing a respective opposing pair of securing mechanisms 803.

In lieu of the elastic member 801, a rack and pinion mechanism 901, such as shown in Fig. 9, or other connecting device may be used to connect the securing mechanisms 803 of opposing dual field generation pads 202. In an embodiment when the joining member is not elastic, it may also have visible distance markings (i.e. mm or cm) or known lengths in order to assure that the dual pads 202 are within a desired maximum distance from each other.

As referenced above, a self-tracking process is performed to determine where the dual field generation pads 202 are co-located. This self-tracking enables the use of the two pads 202 whose physical proximity may change during the course of a medical procedure and/or during the set-up for a medical procedure. This tracking uses the position of the components relative to one another. Each component has at least one transmitter and sensor to communicate with the other components. Each pad 202 includes sensors that read the magnetic field induced by the transmitting coils of the other pad. Such sensors may be incorporated into the field generation units 201, for example, see U.S. Pat. No. 6,618,612. These readings enable one to learn the relative position of the two pads.

The self-tracking algorithm provides the locations and orientations of the transmitting coils in a defined coordinate system. The transmitting coils must have been previously calibrated, that is their magnetic moments (up to any desired order of spherical harmonics) must be known. There are many ways to define the coordinate system. For concreteness, a coordinate system whose origin is the centroid of the location of all the transmitting coils can be chosen. The x-y plane is defined by a plane containing the centers of any two coils of one of the pads 202 and the center of a coil on the other pad 202. In an example of working to order 1 (dipole) in a spherical harmonics expansion, the concept can be readily extended to any order, a rotation matrix can be defined as

$$Rot[\varphi, \theta, \psi]$$
$$= \begin{bmatrix} \cos(\theta)\cos(\phi) & \cos(\theta)\sin(\phi) & -1.\sin(\theta) \\ \cos(\phi)\sin(\theta)\sin(\psi) - 1.\cos(\psi)\sin(\phi) & \cos(\phi)\cos(\psi) + \sin(\theta)\sin(\phi)\sin(\psi) & \cos(\theta)\sin(\psi) \\ \cos(\phi)\cos(\psi)\sin(\theta) + \sin(\phi)\sin(\psi) & \cos(\psi)\sin(\theta)\sin(\phi) - 1.\cos(\phi)\sin(\psi) & \cos(\theta)\cos(\psi) \end{bmatrix}$$

and a dipole polynomial matrix as

$$bDipol[x,y,z] = \begin{bmatrix} \dfrac{-2x^2+y^2+z^2}{(x^2+y^2+z^2)^{5/2}} & -\dfrac{3xy}{(x^2+y^2+z^2)^{5/2}} & -\dfrac{3xz}{(x^2+y^2+z^2)^{5/2}} \\[3mm] -\dfrac{3xy}{(x^2+y^2+z^2)^{5/2}} & \dfrac{x^2-2y^2+z^2}{(x^2+y^2+z^2)^{5/2}} & -\dfrac{3yz}{(x^2+y^2+z^2)^{5/2}} \\[3mm] -\dfrac{3xz}{(x^2+y^2+z^2)^{5/2}} & -\dfrac{3yz}{(x^2+y^2+z^2)^{5/2}} & \dfrac{x^2+y^2-2z^2}{(x^2+y^2+z^2)^{5/2}} \end{bmatrix}$$

Let $(x_i, y_i, z_i)$ be the location of transmitter i and $(x_j, y_j, z_j)$ be the location of transmitter j. Assuming, without loss of generality, that at the center of each coil there is a magnetic sensor, the field measured by the sensor at the center of transmitter i transmitted by coil j can be expressed as:

$$meas_i^j = Rot[\varphi,\theta,\psi]_{sensor\ i}^T\, Rot[\varphi,\theta,\psi]_{transmitter\ j}\, bDipol[Rot[\varphi,\theta,\psi]_{tramsmitter\ j}^T(x_j-x_i, y_j-y_i, z_j-z_i)].\overline{m_j}$$

where $\overline{m_j}$ is the magnetic dipole vector for transmitter j.

This equation has twelve unknowns, six location variables and six orientation variables. A set of N transmitting coils will have a total of nine N unknowns (three location variables and six orientation variables, three for the transmitter and three for the sensor). Each sensor measuring the field from all the transmitters (excluding the one it is associated with) measures 3(N-1) values, for a total of 3 N (N-1) equations.

For any system with four or more coils the number of equations is greater than the number of unknowns. The system can also be solved using an optimization algorithm, with the cost function being the sum (sum of the squares) of the difference between the measurements and the right side of Equation 1. The condition that the origin of the coordinate system is the centroid of the coils centers is included as a constraint. If distances between coils are well known and fixed, they can be included as extra constraints.

It will be appreciated by persons skilled in the art that the present teachings are not limited to what has been particularly shown and described hereinabove. Rather, the scope of the present invention includes both combinations and sub-combinations of the various features described hereinabove, as well as variations and modifications thereof that are not in the prior art, which would occur to persons skilled in the art upon reading the foregoing description.

LIST OF ELEMENTS

| | |
|---|---|
| 24 | patient |
| 28 | support structure |
| 36 | catheter |
| 38 | heart |
| 40 | distal end of catheter |
| 42 | position sensor |
| 44 | console |
| 48 | tracking processor |
| 49 | connector cable |
| 50 | display |
| 100 | magnetic field location system |
| 201 | field generating coil unit |
| 202 | low-profile elongated field generation pad |
| 203 | clamping/securing mechanism |
| 204 | connecting rod |
| 205 | arm rest |
| 206 | securing mechanism arm |
| 207 | adjustable coupling |

208     table engaging arm portion
401     single axis coil
404     connecting rod members
501     clamp handle
502     pivot shaft
503     clamping end portion
801     elastic member
803     securing mechanism
804     boss
806     securing mechanism arm
808     table engaging arm portion
901     rack and pinion mechanism

ASPECTS OF THE INVENTION

1. A method for tracking a distal end of a catheter or the like in a surgical or exploratory procedure, that includes magnetic sensing within a living body disposed on a support structure, comprising:

selectively placing a first field generation pad along a first side of the living body disposed on the support structure, the first field generation pad including a plurality of magnetic coils operable to generate magnetic fields;
selectively placing a second field generation pad along an opposite side of the living body disposed on the support structure, the second field generation pad including a plurality magnetic coils operable to generate magnetic fields;
securing the first and second selectively placed field generation pads to the support structure; and
executing, by a processor coupled to the pluralities of magnetic coils of the first and second field generation pads, a process of correlation of magnetic fields generated by the selectively placed field generation pads such that a reference magnetic field is defined that extends within a desired portion of the living body to enable precise location and tracking of a distal end of a catheter via the magnetic sensor within the reference magnetic field.

2. The method of aspect 1, wherein the selectively placing the first and second field generation defines relatively symmetrical magnetic coil arrays about the sides of the living body.

3. The method of aspect 2 further comprising generating the magnetic fields by first and second field generation pads via two magnetic coils disposed in each field generation pad at a defined distance from each other, where each magnetic coil is configured as a tri-axial coil unit.

4. The method of aspect 3 wherein the generating the magnetic fields is performed by tri-axial coil units that are configured as tri-coil bobbins and have a combined weight of approximately 200 grams.

5. The method of aspect 2 further comprising generating the magnetic fields by the first and second field generation pads via six linearly aligned and equally spaced magnetic coils disposed in each field generation pad, where each magnetic coil is configured as a single coil unit.

6. The method of aspect 1 wherein the first and second field generation pads configured with a height not exceeding 20 mm and a weight not exceeding 3 Kg so that the selectively placing and the securing of the first and second selectively placed field generation pads to the support structure is performed in less than 60 seconds and to enabled the first and second selectively placed field generation pads to be removed from the support structure in less than 60 seconds.

7. The method of aspect 1 wherein:

the securing of the first and second selectively placed field generation pads to the support structure is performed with first and second securing mechanisms attached to each field generation pad at predefined locations spaced apart from each other, where each securing mechanism has an arm having an end attached to the respective field generation pad and a support structure engaging portion; and
the selectively placing the field generation pad on the support structure is performed by engaging the support structure engaging portions with respective sides of the support structure.

8. The method of aspect 7 wherein the securing of the first and second selectively placed field generation pads to the support structure includes operating a clamp of each securing mechanism.

9. The method of aspect 7 further comprising providing an adjustable coupling at the end of the arm of each securing mechanism that is attached to the respective field generation pad, thereby enabling positioning the respective support structure engaging portion at an extended or retracted position relative to the respective field generation pad, whereby the first and second field generation pads are placed furthest from each other and closest to the sides of the support structure when all of the support structure engaging portions are in the retracted position, and whereby the first and second field generation pads are placed closest to each other and furthest from the sides of the support structure when all of the support structure engaging portions are in the extended position.

10. The method of aspect 7, wherein the securing of the first and second selectively placed field generation pads to the support structure includes connecting the first and second securing mechanisms beneath the support structure by at least one joining member.

**Claims**

1. A magnetic field location system for use in tracking a distal end of a catheter or the like, that includes a magnetic sensor, in a surgical or exploratory procedure within a living body disposed on a support structure, comprising:

    a first field generation pad configured for selective placement along a first side of the living body disposed on the support structure that includes a plurality of magnetic coils operable to generate magnetic fields;
    a second field generation pad configured for selective placement along an opposite side of the living body disposed on the support structure that includes a plurality magnetic coils operable to generate magnetic fields;
    a securing structure configured to releasably secure the first and second field generation pads to the support structure when selectively placed on the support structure; and
    a processor coupled to the magnetic coils of the first and second field generation pads configured to execute a process for correlation of magnetic fields generated by the field generation pads such that a reference magnetic field is defined that extends within a desired portion of the living body to enable precise location and tracking of a distal end of a catheter via the magnetic sensor within the reference magnetic field.

2. The magnetic field location system of claim 1, wherein the first and second field generation pads are relatively symmetric and define relatively symmetric magnetic coil arrays when placed by the sides of the living body.

3. The magnetic field location system of claim 2 wherein the magnetic fields generated by the first and second field generation pads are generated by two magnetic coils disposed in each field generation pad at a defined distance from each other, where each magnetic coil is configured as a tri-axial coil unit.

4. The magnetic field location system of claim 3 wherein the tri-axial coil units are configured as tri-coil bobbins and have a combined weight of approximately 200 grams.

5. The magnetic field location system of claim 2 wherein the magnetic fields generated by the first and second field generation pads are generated by six linearly aligned and equally spaced magnetic coils disposed in each field generation pad, where each magnetic coil is configured as a single coil unit.

6. The magnetic field location system of claim 1 wherein each of the first and second field generation pads are configured with a height not exceeding 20 mm and a weight not exceeding 3 Kg and the securing structure is configured such that the first and second field generation pads are able to be selectively placed on and secured to the support structure in less than 60 seconds and are also enable to be removed from the support structure in less than 60 seconds..

7. The magnetic field location system of claim 1 wherein:

    the securing structure includes the first and second securing mechanisms attached to each field generation pad at predefined locations spaced apart from each other;

each securing mechanism having an arm having:

an end attached to the respective field generation pad; and
a support structure engaging portion configured such that the support structure engaging portion engages a side of the support structure when the respective field generation pad is selectively placed on the support structure to thereby define the relative placement of the respective field generation pad on the support structure.

8. The magnetic field location system of claim 7 wherein each securing mechanism has a clamp configured to be operated to a clamping position from an open position to engage the respective support structure engaging portion with a side of the support structure when the respective field generation pad is selectively placed on the support structure.

9. The magnetic field location system of claim 7 wherein the end of the arm of each securing mechanism that is attached to the respective field generation pad has an adjustable coupling to enable the positioning of the respective support structure engaging portion to be positioned at an extended or retracted position relative to the respective field generation pad whereby the first and second field generation pads are placed furthest from each other and closest to the sides of the support structure when all of the support structure engaging portions are in the retracted position, and whereby the first and second field generation pads are placed closest to each other and furthest from the sides of the support structure when all of the support structure engaging portions are in the extended position.

10. The magnetic field location system of claim 7, wherein
the first and second securing mechanisms each have a coupling component configured for connection beneath the support structure; and
the securing structure includes a first joining member configured to attachment to the coupling components of the first securing mechanisms and a second joining member configured for attachment to the coupling components of the second securing mechanisms to secure the selective placement of the field generation pads on the support structure when the joining member are attached.

FIG. 1

FIG. 2A

FIG. 2B

FIG. 2C

FIG. 3

FIG. 4A

FIG. 4B

FIG. 5A

FIG. 5B

202

206

208

203

28

FIG. 6A

206

202

208

503

502

203

501

28

FIG. 6B

206

202

207

501

203

**FIG. 7**

806   202        28        202   806

808                                    808

804              801              804

803                                    803

**FIG. 8A**

FIG. 8B

FIG. 9

Europäisches
Patentamt
European
Patent Office
Office européen
des brevets

# EUROPEAN SEARCH REPORT

**Application Number**

EP 20 17 5066

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 0 883 374 A1 (BIOSENSE INC [US]) 16 December 1998 (1998-12-16) * paragraph [0012] * * figure 1 * ----- | 1-10 | INV. A61B5/06 A61B5/00 A61G13/10 A61G13/12 ADD. A61B34/20 |
| A | US 2017/196477 A1 (WEHNER PETER [US] ET AL) 13 July 2017 (2017-07-13) * paragraphs [0002], [0004], [0020] - [0022], [0027], [0028] * * figures 1-3, 5, 6 * ----- | 1-10 | |
| A | US 2006/025668 A1 (PETERSON THOMAS H [US] ET AL) 2 February 2006 (2006-02-02) * figures 2, 3 * ----- | 1-10 | |
| A | EP 1 570 781 A1 (BIOSENSE WEBSTER INC [US]) 7 September 2005 (2005-09-07) * figure 6 * ----- | 1-10 | |

**TECHNICAL FIELDS SEARCHED (IPC)**

A61B

The present search report has been drawn up for all claims

| Place of search | Date of completion of the search | Examiner |
|---|---|---|
| The Hague | 17 September 2020 | Worms, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 17 5066

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2020

| Patent document cited in search report | | | Publication date | Patent family member(s) | | Publication date |
|---|---|---|---|---|---|---|
| EP 0883374 | A1 | | 16-12-1998 | AU | 706052 B2 | 10-06-1999 |
| | | | | CA | 2246343 A1 | 21-08-1997 |
| | | | | DE | 69733604 T2 | 11-05-2006 |
| | | | | DE | 69738274 T2 | 28-08-2008 |
| | | | | EP | 0883374 A1 | 16-12-1998 |
| | | | | EP | 1481635 A2 | 01-12-2004 |
| | | | | ES | 2243976 T3 | 01-12-2005 |
| | | | | ES | 2295767 T3 | 16-04-2008 |
| | | | | HK | 1070259 A1 | 14-03-2008 |
| | | | | IL | 125760 A | 31-07-2003 |
| | | | | JP | 3881028 B2 | 14-02-2007 |
| | | | | JP | 2000507845 A | 27-06-2000 |
| | | | | US | 6366799 B1 | 02-04-2002 |
| | | | | WO | 9729683 A1 | 21-08-1997 |
| US 2017196477 | A1 | | 13-07-2017 | CN | 108778117 A | 09-11-2018 |
| | | | | EP | 3386386 A2 | 17-10-2018 |
| | | | | JP | 6641016 B2 | 05-02-2020 |
| | | | | JP | 2019511256 A | 25-04-2019 |
| | | | | JP | 2020089739 A | 11-06-2020 |
| | | | | US | 2017196477 A1 | 13-07-2017 |
| | | | | US | 2020085342 A1 | 19-03-2020 |
| | | | | WO | 2017123483 A2 | 20-07-2017 |
| US 2006025668 | A1 | | 02-02-2006 | NONE | | |
| EP 1570781 | A1 | | 07-09-2005 | AT | 442082 T | 15-09-2009 |
| | | | | EP | 1570781 A1 | 07-09-2005 |

EPO FORM P0459

For more details about this annex : see Official Journal of the European Patent Office, No. 12/82

**EP 3 730 051 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 62591238 A
- US 6618612 B
- US 5391199 A
- US 6690963 B
- US 6484118 B
- US 6239724 B
- US 6332089 B
- WO 9605768 A
- US 20020065455 A1
- US 20030120150 A1
- US 20040068178 A1